(19) Europäisches Patentamt
European Patent Office
Office européen des brevets

(11) Veröffentlichungsnummer: **0 501 251 A2**

(12) # EUROPÄISCHE PATENTANMELDUNG

(21) Anmeldenummer: **92102450.1**

(51) Int. Cl.5: **C07C 217/84**

(22) Anmeldetag: **14.02.92**

(30) Priorität: **27.02.91 US 661720**

(43) Veröffentlichungstag der Anmeldung: **02.09.92 Patentblatt 92/36**

(84) Benannte Vertragsstaaten: **AT BE CH DE DK ES FR GB GR IT LI LU NL SE**

(71) Anmelder: **BAYER AG**
**W-5090 Leverkusen 1 Bayerwerk(DE)**

(72) Erfinder: **Lenfers, Jan-Bernd, Dr.**
**Hubertusallee 9**
**W-5600 Wuppertal 1(DE)**
Erfinder: **Muschalek-Letina, Volker, Dr.**
**Claudiusweg 3**
**W-5600 Wuppertal 1(DE)**
Erfinder: **Niemers, Ekkehard, Dr.**
**In den Birken 51a**
**W-5600 Wuppertal 1(DE)**
Erfinder: **Janis, Ronald A., Dr., Miles Inc.**
**Pharmaceutical Division, 400 Morgan Lane**
**West Haven, CT 06516(US)**
Erfinder: **Scriabine, Alexander, Dr., Miles Inc.**
**Pharmaceutical Division, 400 Morgan Lane**
**West Haven, CT 06516(US)**

(54) **Enantiomer ( + )N-(2-Ethoxyphenyl)-N',N'-(1,2,2-trimethylpropyl)-2-nitroethen-1,1-diamin, Verfahren zu seiner Herstellung und seine Verwendung.**

(57) ( + )-N-(2-Ethoxyphenyl)-N',N'-(1,2,2-trimethylpropyl)-2-ni troethen-1,1-diamin kann durch Umsetzung von 1-[-(1-Ethoxy)phenylamino]-1-methylthio-2-nitro-ethen mit entweder racemischem 1,2,2-Trimethylpropargylamin sowie anschließende chromatographische Abtrennung des ( + )-enantiomeren Endprodukts, oder direkte Umsetzung mit enantiomeren reinem (1,2,2)-Trimethylpropargylamin hergestellt werden. Das ( + )-N-(2-Ethoxyphenyl)-N',N'-(1,2,2-trimethylpropyl)-2-nitro-ethen-1,1-diamin kann als Wirkstoff in Arzneimitteln zur Behandlung von Hypertension, Herzrhythmusstörungen, Bronchialasthma oder cerebralen Erkrankungen verwendet werden.

EP 0 501 251 A2

Die Erfindung betrifft das Enantiomer (+)-N-(2-Ethoxyphenyl)-N'-(1,2,2-trimethylpropyl)-2-nitroethen-1,1-diamin, Verfahren zu seiner Herstellung und seine Verwendung als kreislaufbeeinflussendes und muskelrelaxierendes Arzneimittel, insbesondere als blutdrucksenkendes Mittel.

Bei einem Racemat ist die pharmakologische Wirkung manchmal nur an eines der beiden Enantiomere gebunden ist, während das andere Isomere keine oder nur eine geringe oder eine entgegengesetzte Wirkung hat und somit entweder als Ballast mitgeschleppt wird oder sogar für unerwünschte Nebenwirkungen verantwortlich ist.

In der DE 32 32 462 wird das Racemat N-(2-Ethoxyphenyl)-N',-(1,2,2-tiimethylpropyl)-2-nitroethen-1,1-diamin mit einer kreislaufbeeinflussenden Wirkung beschrieben.

Durch Untersuchungen an $K^+$-Kanälen bezüglich der Membranmodulation lassen sich interessante Wirkungen auffinden, die bei der Suche nach Arzneimitteln nützlich sind. Während die $K^+$-Kanal-Blocker die Insulinsekretion steigern und Dysrhythmien hemmen, können die $K^+$-Kanal-Aktivatoren aufgrund ihrer relaxierenden Wirkung auf die glatte Muskulatur bei einer großen Bandbreite von Erkrankungen, wie beispielsweise Hypertension, Bronchialasthma oder Reizblase eingesetzt werden [vgl. Nigel S. Cook, TIPS, Jan. 1988 (Vol. 9), S. 22-28; A.H. Weston, DNP 1(4), Sept. 1988, 205-209].

Die Erfindung betrifft das Enantiomer (+)-N-(2-Ethoxyphenyl)-N'-(1,2,2-trimethylpropyl)-2-nitroethen-1,1-diamin der Formel (I).

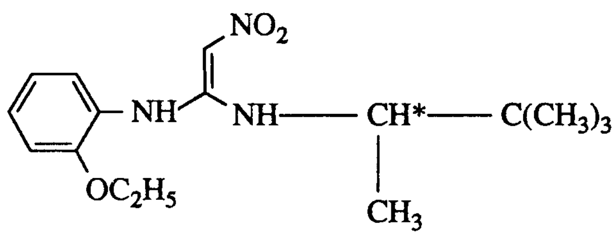

(I)

(+)-Enantiomer

Die erfindungsgemäße Verbindung ist neu und besitzt wertvolle pharmakologische Eigenschaften. Überraschenderweise zeichnet sie sich durch eine außerordentlich starke kreislaufbeeinflussende und die glatte Muskulatur relaxierende Wirkung aus.

Die erfindungsgemäße Verbindung kann in Form des reinen E- oder Z-Isomers oder als E/Z-Isomerengemisch vorliegen. Unter physiologischen Bedingungen wird in Analogie zu literaturbekannten Diaminonitroethylenen eine Gleichgewichtseinstellung des E- und Z-Isomeres beobachtet [vgl. Mag. Res. Chem. 25, 205-207 (1987); Gaz. Chim. Hal.,111, 217 (1981); Acta Chem. Scand. 27, 1183 (1973)].

Die erfindungsgemäße Verbindung der Formel (I) kann hergestellt werden, indem man

[A] 1-[(1-Ethoxy)phenylamino]-1-methylthio-2-nitro-ethen der Formel (II)

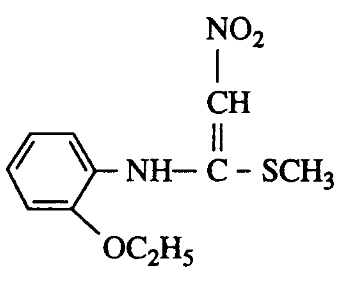

(II)

mit 1,2,2-Trimethylpropylamin der Formel (III)

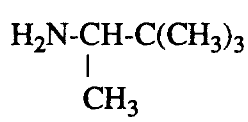

(III)

gegebenenfalls in Anwesenheit eines organisches Lösemittels, zu dem entsprechenden Racemat umsetzt und anschließend dieses direkt an chiraler stationärer Phase mit High-Performance Liquid Chromatography

(HPLC) und organischen Lösemitteln das (+)-Enantiomere abtrennt,
oder indem man
[B] das enantiomerenreine 1,2,2-Trimethylpropylamin der Formel (IIIa)

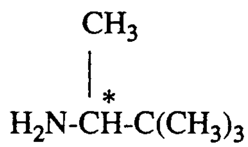

(IIIa)

mit [(1-Ethoxy)phenylamino]-1-methylthio-2-nitro-ethen der Formel (II) nach der unter A angegebenen Methode umsetzt.

Die Verfahren können durch folgendes Formelschema beispielhaft erläutert werden:

[A]

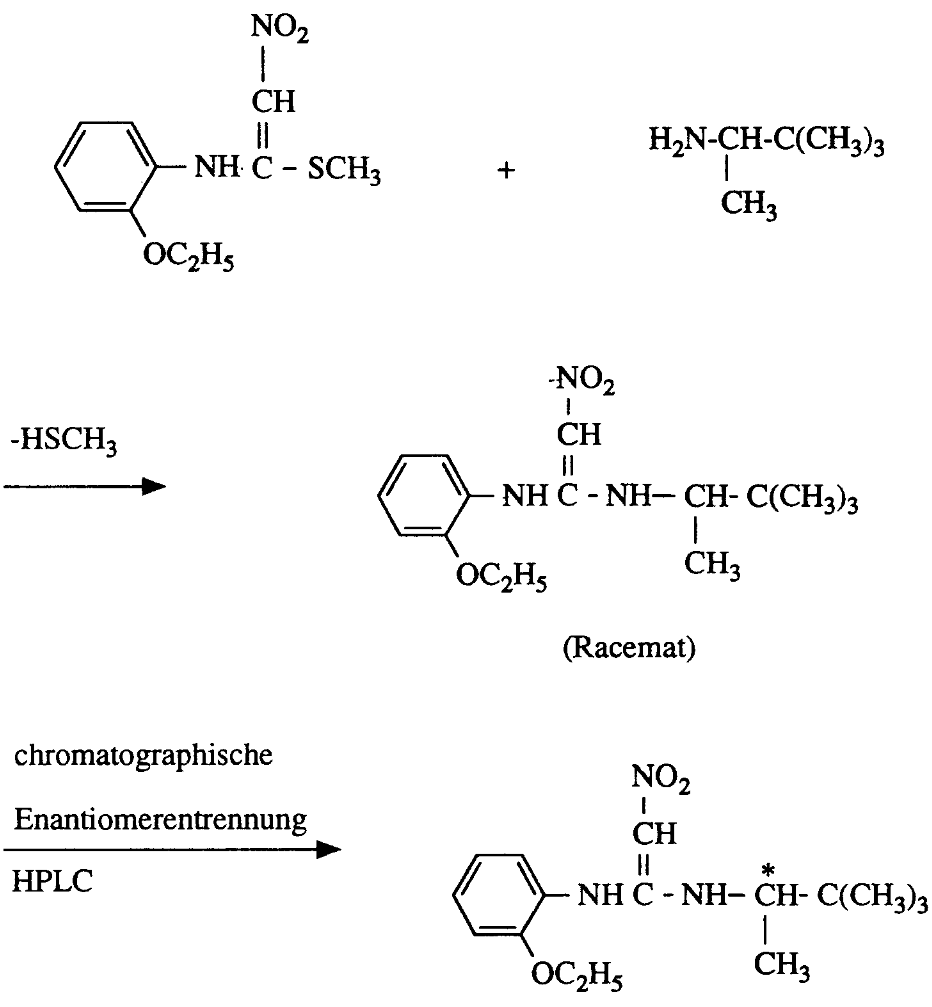


(Racemat)

(+)-Enantiomer
(-)-Enantiomer

[B]

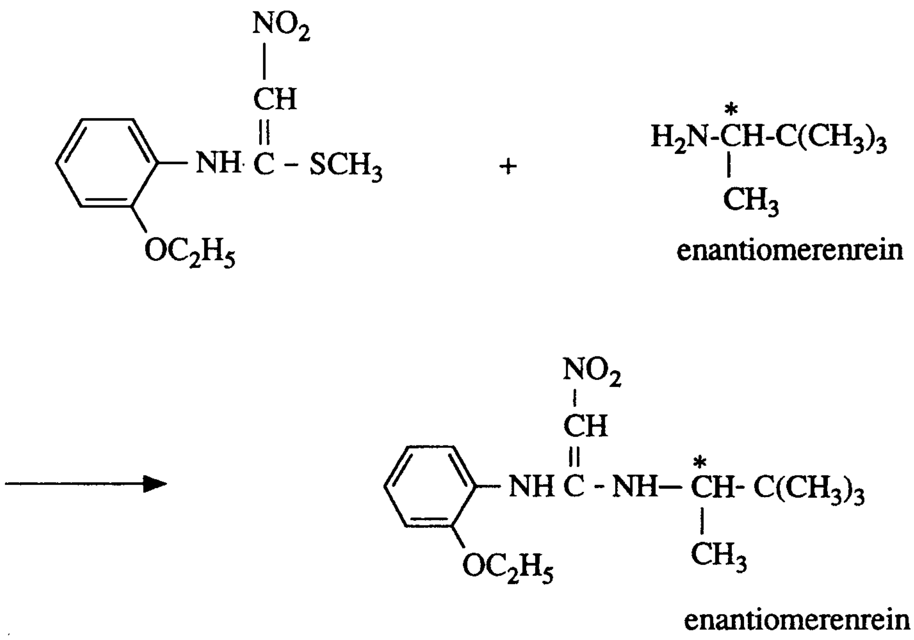

Die Darstellung des Racemats ist in der DE 32 32 462 beschrieben.

Im Fall, daß die Umsetzung in Anwesenheit eines organischen Lösemittels durchgeführt wird, eignen sich als organische Lösemittel Kohlenwasserstoffe wie Hexan, Toluol, Xylol, oder Chlorkohlenwasserstoffe wie Methylenchlorid, Chloroform, oder Ether wie Diethylether, Dioxan, Tetrahydrofuran oder Alkohole wie Ethanol, Ethylenglykol oder Methanol, sowie Eisessig, Pyridin, Dimethylsulfoxid oder Dimethylformamid. Außerdem ist eine Reaktionsdurchführung ohne Lösemittel, aber mit einem Überschuß des Amins 1,2,2-Trimethylpropylamin möglich. Bevorzugt ist der Einsatz von Eisessig, Ethanol sowie der Überschuß des Amins.

Die Umsetzung wird in einem Temperaturbereich von 50 °C bis 160 °C, bevorzugt von 50 °C bis 120 °C durchgeführt.

Im allgemeinen wird die chemische Umsetzung bei Normaldruck durchgeführt. Die HPLC-Trennung wird mit einem Überdruck von 10 - 25 bar durchgeführt.

Die Verbindung der Formel (III) wird mit 1 - 5 Mol, bevorzugt mit 1 - 3 Mol, bezogen auf 1 Mol der Verbindung (II) umgesetzt.

Bei der HPLC-Trennung werden sowohl die präparativen als auch die analytischen Arbeiten mit einer Phase aus Cellulose-(tris-3,5-dimethylphenylcarbamat) auf Kieselgel, Handelsname Chiracel OD durchgeführt.

Als Lösemittel eignen sich dabei Kohlenwasserstoffe wie n-Heptan und n-Hexan, oder Alkohole wie 2-Propanol. Es ist außerdem möglich, Gemische dieser Lösemittel zu verwenden. Bevorzugt sind die Gemische n-Hexan/2-Propanol 78:22 für den präparativen und n-Heptan/2-Propanol 75:25 für den analytischen Teil der Chromatographie.

Die HPLC wird bei 20 °C bis 60 °C, vorzugsweise bei 40 °C durchgeführt.

Ausgehend von der Racematform ist die Auftrennung in die stereochemisch einheitlichen Bestandteile, insbesondere das erfindungsgemäße (+)-Enantiomer grundsätzlich auch nach anderen literaturbekannten Methoden möglich [vgl. E.L. Eliel, Stereochemistry of Carbon Compounds, McGraw Hill, 1962].

Die Verbindungen der Formeln (II), (III) und (IIIa) sind bekannt und können nach literaturbekannten Methoden hergestellt werden [Chem. Ber. 100, 591 - 604 (1967); Houben-Weyl XI/1 (1957); H.E. Smith, E.E. Ensley, Can. J. of Chem. 49, 1971, 2902].

Die erfindungsgemäße Verbindung beeinflußt die Kontraktionskraft des Herzens und wirkt außerdem relaxierend auf die glatte Muskulatur der Gefäße. Sie kann deshalb in Arzneimitteln zur Behandlung des pathologisch veränderten Blutdrucks (Hypertension), von Herzrhythmusstörungen und von Bronchlalasthma eingesetzt werden. Darüberhinaus kann sie zur Behandlung von cerebralen Erkrankungen verwendet

werden.

Die durch K-Kanal verursachte Vasorelaxation wird wie folgt geprüft:
Männliche Long-Evans Ratten (200 - 225 g) werden durch Enthauptung getötet. Die Thorax-Aorta wird sofort entnommen und sofort in eine mit Sauerstoff (100% $O_2$) gesättigte physiologische Salzlösung (PSL) (pH 7,4), bestehend aus 130 nM NaCl, 5,9 nM KCl, 1,2 nM $MgCl_2$, 0,8 nM $CaCl_2$, 20 nM HEPES und 11,1 nM Glucose, gegeben.

Die Aorta wird vom Fett gesäubert und in 3 - 4 mm breite Ringe geschnitten. Jeder Ring wird in ein Organbad, das 10 ml der sauerstoffgesättigten PSL enthält, gegeben und unter einer Spannung von 2 g eingespannt. Die isometrische Spannung wird an einem Beckman R6 11-Dynographen mit einem Dehnungsmeßstreifen - Kraftaufnehmer vom Typ S 872 und einem Grass FT 03 D Transducer gemessen. Die Ringe werden 90 min equilibriert, wobei jeweils nach 20 - 30 min die PSL gewechselt wird. Gegebenenfalls wird am Ende einer Equilibrierungsperiode die Spannung auf 2 g rejustiert. Die Kontraktion der Ringe wird durch eine 20 mM Steigerung der Konzentration von KCl in PSL induziert. Die Ringe werden 2 mal in 20 mM KCl zur maximalen Kontraktion gebracht. Nachfolgende Kontraktionen für den Test werden induziert und etwa 20 min nach KCl-Addition, wenn die Kontraktion ein Plateau erreicht, wird die gewünschte Substanzkonzentration in das Bad gegeben. Die Inhibition der Kontraktion durch die erfindungsgemäße Verbindung wird durch den Sulfonylharnstoff Glyburid, einem bekannten Antagonisten [Br. J. Pharmacol., 97, 57-64, J. Pharm. Exp. Ther. 248, 1261 - 1268, 149-156] des ATP-abhängigen $K^+$-Kanals aufgehoben.

| Beispiel | $IC_{50}$ (nM) |
|---|---|
| 1 | 15,5 |

Das entsprechende (-)-Enantiomer (Beispiel 2) ist mit einem $IC_{50}$-Wert von 248 (nM) unwirksam.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Das Racemat (-/+)-N-(2-Ethoxyphenyl)-N'-(1,2,2-trimethylpropyl)-2-nitroethen-1,1-diamin, dessen Herstellung in der DE 32 32 462 beschrieben ist, wird direkt an chiraler stationärer Phase mit High-Performance-Liquid Chromatography (HPLC) getrennt.

Verwendete Materialien

1. Stationäre Phase:

Für die präparativen Arbeiten wird Cellulose-(tris-3,5-dimethylphenylcarbamat) auf Kieselgel adsorbiert,

Handelsname: Chiracel OD, erhältlich bei Firma J. T. Baker, (Teilchengröße: 20 µ, Porenweite: 1000 Å). Das Material wird mit Slurry-Technik in die Säule gefüllt.
Die analytischen Arbeiten werden auf der oben angegebenen Phase gemacht. Die Fertigsäule (Teilchengröße 10 µ) wurde bei J.T. Baker gekauft.

2. Lösungsmittel

n-Heptan, p.A. (Merck)
n-Hexan, p.A. (Merck)
2-Propanol, p.A. (Merck)

Probenvorbereitung:

300 mg Racemat (-/+)-N-(2-Ethoxyphenyl)-N'-(1,2,2-trimethylpropyl)-2-nitroethen-1,1-diamin werden in 40 ml 2-Propanol gelöst.

Chromatographiebedingungen:

präparativ:

| | |
|---|---|
| Säule: | Länge 550 mm<br>Innendurchmesser: 20 mm |
| Fluß: | 10 ml/min |
| Fließmittel: | n-Hexan/2-Propanol 78/22 |
| Laufzeit einer Injektion: | 45 min |
| Detektion: | UV-Vis-Detektor,<br>Wellenlänge: 330 nm |
| Temperatur: | 40 °C |
| Aufgabevolumen: | 1 ml = 7,5 mg |

analytisch:

| | |
|---|---|
| Säule: | Länge: 250 mm<br>Innendurchmesser: 4,6 mm |
| Fließmittel: | n-Heptan/2-Propanol 75/25 |
| Fluß: | 1 ml/min. |
| Detektion: | UV-Vis-Detektor<br>Wellenlänge: 230 nm |
| Temperatur: | 40 °C |
| Aufgabevolumen: | 20 µl |

Beispiel 1

(+)-N-(2-Ethoxyphenyl)-N'-(1,2,2-trimethylpropyl)-2-nitroethen-1,1-diamin

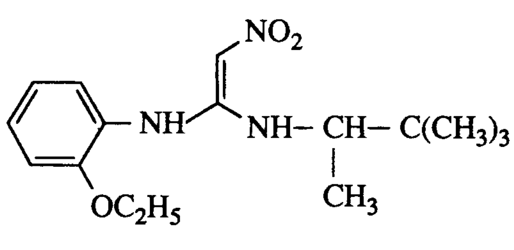

(+)-Enantiomer

Ausbeute: 100 mg
ee > 99%
$[\alpha]^{20}_D$ = +63,1 ° (c = 0,94, $CH_2Cl_2$)
Retentionszeit: 13,3 min

Beispiel 2 (Vergleichsverbindung)

(-)-N-(2-Ethoxyphenyl)-N'-(1,2,2-trimethylpropyl)-2-nitroethen-1,1-diamin
Ausbeute: 100 mg
ee > 99%
$[\alpha]^{20}_D$ = -61,3° (c = 0,9, $CH_2Cl_2$)
Retentionszeit: 14,3 min

**Patentansprüche**

1. (+)-N-(2-Ethoxyphenyl)-N'-(1,2,2-trimethylpropyl)-2-nitro-ethen-1,1-diamin der Formel (I)

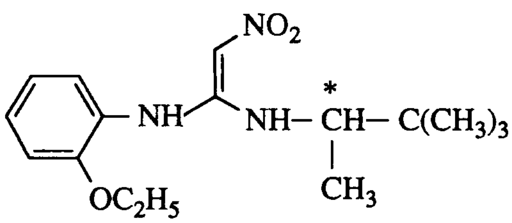

(+)-Enantiomer (I)

2. (+)-N-(2-Ethoxyphenyl)-N'-(1,2,2-trimethylpropyl)-2-nitroethen-1,1-diamin zur Behandlung von Krankheiten.

3. Verfahren zur Herstellung von(+)-N-(2-Ethoxyphenyl)-N'-(1,2,2-trimethylpropyl)-2-nitroethen-1,1-diamin der Formel

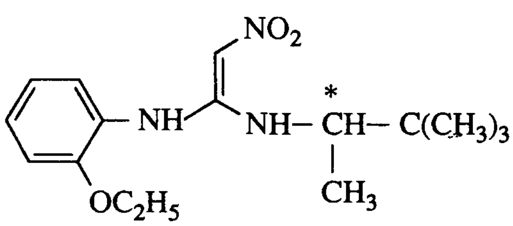

(+)-Enantiomer (I)

dadurch gekennzeichnet, daß man

[A] 1-[(1-Ethoxy)phenylamino]-1-methylthio-2-nitro-ethen der Formel (II)

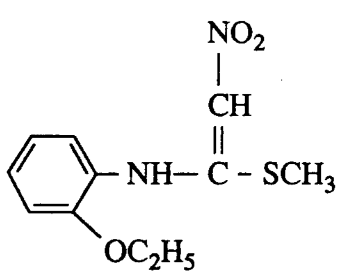

(II)

mit 1,2,2-Trimethylpropylamin der Formel (III)

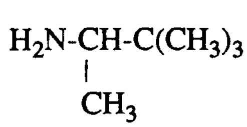

(III)

gegebenenfalls in Anwesenheit eines organisches Lösemittels, zu dem entsprechenden Racemat

umsetzt und anschließend dieses direkt an chiraler stationärer Phase mit High-Performance Liquid Chromatography (HPLC) und organischen Lösemitteln das (+)-Enantiomere abtrennt,

oder daß man

[B] das enantiomerenreine 1,2,2-Trimethylpropylamin der Formel (IIIa)

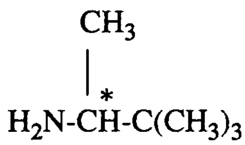

(IIIa)

mit [(1-Ethoxy)phenylamino]-1-methylthio-2-nitro-ethen der Formel (II) nach der unter A angegebenen Methode umsetzt.

4. Verfahren nach Anspruch 3, dadurch gekennzeichnet, daß die Umsetzung in einem Temperaturbereich von 50 °C bis 160 °C durchgeführt wird.

5. Arzneimittel enthaltend (+)-N-(2-Ethoxyphenyl)-N'-(1,2,2-trimethylpropyl)-2-nitro-ethen-1,1-diamin.

6. Verfahren zur Herstellung eines Arzneimittels nach Anspruch 5, dadurch gekennzeichnet, daß das (+)-N-(2-Ethoxyphenyl)-N'-(1,2,2-trimethylpropyl)-2-nitro-ethen-1,1-diamin gegebenenfalls mit üblichen Hilfs- und Trägerstoffen in eine geeignete Applikationsform überführt.

7. Arzneimittel nach Anspruch 6, enthaltend den Wirkstoff in einer Menge von 0,5 bis 90 Gew.-% der Gesamtmenge.

8. Verwendung von (+)-N-(2-Ethoxyphenyl)-N'-(1,2,2-trimethylpropyl)-2-nitro-ethen-1,1-diamin zur Herstellung von Arzneimitteln.

9. Verwendung nach Anspruch 7 zur Herstellung von Arzneimitteln zur Behandlung von Hypertension, Herzrhythmusstörungen, Bronchialasthma sowie cerebralen Erkrankungen.